**Europäisches Patentamt**

**European Patent Office**

**Office européen des brevets**

(19)

(11) Publication number: **0 235 482**
**A1**

(12)
# EUROPEAN PATENT APPLICATION

(21) Application number: **86402932.7**

(22) Date of filing: **24.12.86**

(51) Int. Cl.⁴: **A 61 L 33/00**, A 61 L 29/00,
C 08 F 283/00
// (C08F283/00, 214:06)

(30) Priority: **28.12.85 JP 297969/85**

(43) Date of publication of application: **09.09.87**
**Bulletin 87/37**

(84) Designated Contracting States: **BE DE FR GB IT SE**

(71) Applicant: **TERUMO KABUSHIKI KAISHA trading as TERUMO CORPORATION, 44-1, 2-chome, Hatagaya Shibuya-Ku, Tokyo 151 (JP)**

(72) Inventor: **Ohachi, Yoshinuri, 936 Yodohira-cho, Fujinomiya-shi Shizuoka-ken (JP)**
Inventor: **Ishikawa, kunihiro, 27-12, 1-chome, Nakano Nakano-ku, Tokyo (JP)**
Inventor: **Endo, Fumiaki, 19-10, 4-chome, Yoshiwara, Fuji-shi Shizuoka-ken (JP)**
Inventor: **Nagai, Hirofumi, Terumo K.K. Fujimi-Ryo, 2517 Ohmiya Fujinomya-shi Shizuoka-ken (JP)**

(74) Representative: **Descourtieux, Philippe et al, CABINET BEAU de LOMENIE 55 rue d'Amsterdam, F-75008 Paris (FR)**

(54) **Antithrombotic medical material and intravascular catheter made of said material.**

(57) Antithrombotic material for medical use, which comprises a resin prepared by graft-copolymerizing vinyl chloride with polyurethane resin. The graft-copolymerised resin preferably contains 10 to 80% by weight of polyurethane elastomer. There are also proposed an intravascular indwelling catheter (1) made of the resin described above, and a method of inhibiting the formation of thrombus on a surface of a medical instrument such as a catheter while it is indwelled in a blood vessel.

EP 0 235 482 A1

- 1 -

Antithrombotic medical material and
intravascular catheter made of said material

The present invention relates to an antithrombotic
material and an intravascular catheter made of the
antithrombotic material and, more particularly, to an
intravascular catheter such as intravenous indwelling
catheters and intra-arterial indwelling catheters having
a good antithrombotic property.

Various types of intravascular indwelling catheters
have been proposed and used in practice.  For example,
conventional intravenous indwelling catheters include
an IVH (Intravenous Holoneutrition) catheter and a CVP
(Central Venous Pressure measuring) catheter, and
conventional intra-arterial indwelling catheters include
an angiographic catheter and an arterial pressure
measuring catheter.  Most of these catheters are made
of a soft vinyl chloride resin plasticized with DEHP
(diethylhexylphthalate).  However, when an intravascular
indwelling catheter made of the above-mentioned resin
is indwelled in a blood vessel of a patient for a long
period of time, DEHP may elute in the blood, thus
endangering the patient.  In addition, after elution
occurs, the resin is hardened to lose necessary physical
properties of the catheter.  In this state, the
antithrombotic property is lost, and blood aggregation
and conglutination occur on the surface of the catheter
to obstruct blood flow in small blood vessels or a separated aggregate forms an agglutinative thrombus.

Another conventional catheter made of polyamide is also available. However, this catheter has the same problem of blood aggregation as in the catheter made of the soft vinyl chloride resin.

In order to prevent the blood aggregation, a method is proposed to modify the surface of the catheter with an antithrombotic material (e.g., heparin and urocanase) derived from a living organism. However, the fabrication of such a catheter is difficult, the heat-resistant property in the sterilization process is degraded, and the antithrombotic property is degraded again upon separation of the modifier from the surface of the catheter.

It is an object of the present invention to provide an antithrombotic material for medical use which can solve the conventional problems described above, which has a good antithrombotic property, and which is free from change in physical properties for a long period of time.

Another object of the present invention is to provide an intravascular catheter such as IVH catheter, CVP catheter, angiographic catheter and arterial pressure measuring catheter which can solve the conventional problems described above, which has a good antithrombotic property, and which is free from change in physical properties for a long period of time.

In order to achieve the above objects of the present invention, there are provided an antithrombotic material for medical use, which comprises a resin prepared by graft-copolymerizing vinyl chloride with polyurethane resin, and an intravascular catheter comprising a tube having an outer diameter of 2.0 mm or less and made of a resin prepared by graft-copolymerizing vinyl chloride with polyurethane resin.

The graft-copolymerized resin is preferably prepared by graft-copolymerizing a vinyl chloride monomer or a monomer mixture consisting of a vinyl chloride monomer and a monomer copolymerizable therewith so as to produce a homopolymer having a glass transition point of less than 30°C, in an aqueous medium, in the presence of a thermoplastic polyurethane elastomer soluble in the vinyl chloride monomer and having a softening point of 20 to 100°C.

It is preferable that the graft-copolymerized resin contains 10- to 80 % by weight of polyurethane elastomer.

In the graft copolymerization reaction, 10 to 200 parts by weight of the thermoplastic polyurethane elastomer are preferably employed for 100 parts by weight of the vinyl chloride monomer or the monomer mixture.

The figure is a plan view of an intravascular indwelling catheter according to an embodiment of the present invention.

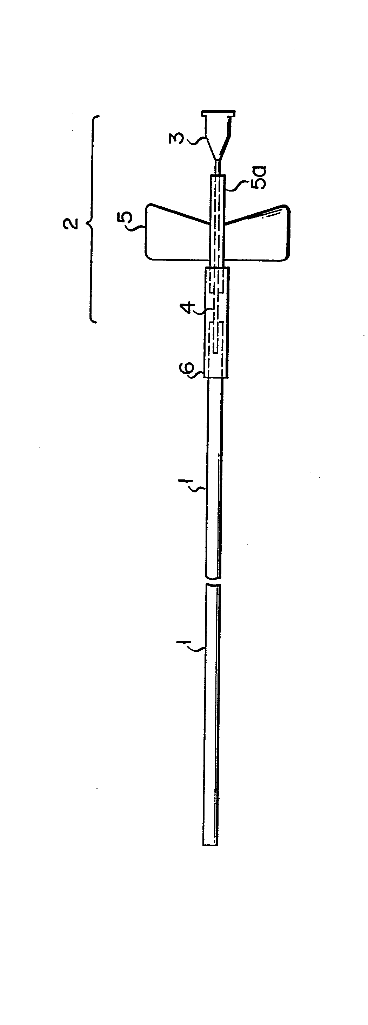

Intravascular indwelling catheters can be exemplified by an IVH catheter, a CVP catheter, an angiographic catheter, an arterial pressure measuring catheter, and the like. The present invention will be described with reference to an IVH catheter shown in Fig. 1.

The IVH catheter in Fig. 1 comprises a tube body and fixing member 2. The tube body constitutes intravascular indwelling catheter 1. Fixing member 2 is integrally coupled to the rear end of the tube body to fix catheter 1 on the skin surface of a patient. Fixing member 2 comprises hub 3, catheter connecting tube 4 fixed to hub 3, wing 5 having body 5a fitted on tube 4, and protective tube 6 fitted on the distal end of body 5a of wing 5 to connect catheter 1 to tube 4. Catheter 1 is made of a resin (to be referred to as a copolymer resin hereinafter) prepared by graft-copolymerizing vinyl chloride with polyurethane resin. More specifically, the copolymer resin is prepared by polymerizing a vinyl chloride monomer or a monomer mixture (to be referred to as a vinyl chloride monomer mixture hereinafter) consisting of a vinyl chloride monomer and a monomer copolymerizable therewith to prepare a homopolymer having a glass transition point of less than 30°C. This polymerization is performed in an aqueous medium in the presence of 10 to 200 parts by weight (preferably 10 to 100 parts by weight) of a thermoplastic polyurethane elastomer (to be referred to as a polyurethane elastomer hereinafter) with respect to 100 parts by weight of the vinyl chloride monomer or the vinyl chloride monomer mixture. The polyurethane elastomer is soluble in the vinyl chloride monomer and has a softening point of 20 to 100°C.

The copolymer resin is prepared by polymerizing the vinyl chloride monomer or the vinyl chloride monomer mixture while the polyurethane elastomer is dissolved in the vinyl chloride monomer or the vinyl chloride monomer mixture. The mechanism of this reaction has not been determined. However, it is assumed that graft copolymerization occurs between the polyurethane elastomer and the vinyl chloride monomer or the vinyl chloride monomer mixture. This is supported by the fact that the graft copolymer resin has better workability and higher transparency and flexibility than the polyurethane elastomer and the vinyl chloride before they are mixed. The polyurethane elastomer used in the preparation of the copolymer resin is substantially dissolved in the vinyl chloride monomer or the vinyl chloride monomer mixture. The polyurethane elastomer has a softening point of 20 to 100°C, and more preferably 30 to 60°C. If the softening point exceeds 100°C, the elastomer tends not to be dissolved in the vinyl chloride monomer. If the softening point is lower than 20°C, tensile strength and heat resistance of the resultant copolymer resin are degraded. A preferable polyurethane elastomer is of a non-yellowing type using aliphatic diisocyanate. A non-yellowing elastomer has stability against ultraviolet rays, while a yellowing elastomer causes coloring of the resultant copolymer resin.

The polyurethane elastomer used in the copolymer resin for the catheter of the present invention mainly contains a polyesterdiol or a polyetherdiol and aliphatic diisocyanate. An example of polyesterdiol is adipic acid polyesterdiol. Examples of the aliphatic diisocyanate are tetramethyleneisocyanate, pentamethylenediisocyanate, and hexamethylenediisocyanate. The viscosity of a mixture obtained by mixing 20% of a polyurethane elastomer into methyl ethyl ketone is preferably 30 to 1,000 cps, more preferably 50 to 400 cps, and most preferably 100 to 300 cps.

Examples of the polyurethane elastomer are Pandex T-5265 and Pandex T-525 available from DAINIPPON INK & CHEMICALS, INC.

In the polymeric resin used for the catheter according to the present invention, 10 to 200 parts by weight, and preferably 10 to 100 parts by weight of the polyurethane elastomer are used with respect to 100 parts by weight of the vinyl chloride monomer or the vinyl chloride monomer mixture at the time of polymerization.

If the content of the polyurethane elastomer is less than 10 parts by weight with respect to 100 parts by weight of the vinyl chloride monomer or the vinyl chloride monomer mixture, the resultant copolymer resin does not have satisfactory softness. However, if the content exceeds 200 parts by weight, a polymerization rate is undesirably reduced.

If the content falls within the range between 10 and 100 parts by weight, suitable physical properties of the catheter can be obtained at the time of insertion and indwelling of the catheter in the blood vessel.

The content of the polyurethane elastomer in the copolymer resin prepared in the manner as described above is preferably 10 to 80% by weight, and more preferably 17 to 65% by weight. If the content is less than 10% by weight, satisfactory softness cannot be obtained. However, if the content exceeds 80% by weight, heat resistance of the resultant resin is degraded, and the preparation cost is undesirably increased.

In the copolymer resin used in the present invention, examples of the monomer copolymerizable with the vinyl chloride monomer to prepare a homopolymer having a glass transition point of 30°C or less are olefins (e.g., ethylene and propylene), vinylidene halides (e.g., vinylidene chloride), vinyl esters (e.g., vinyl acetate), vinyl ethers (e.g., n-butylvinyl ether), acrylic esters (e.g., acrylic butyl, acrylic 2-ethylhexyl), and methacrylic esters (e.g., methacrylic acid-2-ethylhexyl). The content of the above-mentioned monomer in the mixture of the vinyl chloride monomer mixture is preferably 50% by weight or less, and more preferably 30% by weight or less. If the content exceeds 50% by weight, workability, heat resistance, and transparency of the resultant copolymer resin are degraded.

A suspension agent used in the preparation of the copolymer resin used in the present invention may be a known one. Examples are partially saponified polyvinyl alcohol, methyl cellulose, ethyl cellulose, hydroxymethyl cellulose, polyacrylic acid, a vinyl ether-maleic anhydride copolymer, and gelatine. These suspension agents may be used singly or in a combination of more than one.

The amount of the suspension agent is 0.01 to 2% by weight with respect to the aqueous medium.

An oil-soluble polymerization initiator used for preparing the copolymer resin used in the present invention may be a known initiator. Examples are an azo compound (e.g., azobisisobutylvaleronitrile), and an organic oxide (e.g., lauryl peroxide, di-2-ethylhexylperoxydicarbonate, and t-butylperoxypivalate). The amount of the oil-soluble polymerization initiator is 0.01 to 2% by weight with respect to the vinyl chloride monomer mixture when it is mixed. A ratio of aqueous medium to the mixture of the polyurethane elastomer and the vinyl chloride monomer mixture is 1/1 to 3/1. If the ratio is less than 1/1, polymerization is unstable. However, if the ratio exceeds 3/1, an economical disadvantage results in. The polymerization temperature is 30 to 70°C, and preferably 40 to 60°C. If the temperature is lower than 30°C, the polymerization rate is reduced. However, if the temperature exceeds 70°C, heat resistance of the copolymer resin is degraded.

A known chain transfer agent such as trichloroethylene or mercaptoethanol may be used in the preparation of the copolymer resin used in the present invention. Suspension polymerization is normally used, but emulsion polymerization may be utilized.

In order to prevent thermal aging of the soft thermoplastic resin, a stabilizer may be added to the copolymer resin used in the present invention. In this case, a toxic material such as lead- or cadmium-based stabilizer must not be used as the stabilizer. Examples of the usable stabilizer are calcium stearate and zinc stearate. Epoxidized soybean oil may also be used, provided the amount thereof is limited to a very small amount. In addition, a lubricant or the like can be used within the range free from toxicity. A copolymer resin containing a stabilizer or the like can be molded and worked in a press molding machine, an extrusion molding machine, an injection molding machine, a blow molding machine, an inflation molding machine, a calendering machine or the like, which is conventionally used. The copolymer resin is soft and can be thermally welded by an RF welding machine or adhered by a solvent.

The tube body constituting catheter 1 according to the present invention can be easily prepared by extrusion-molding the copolymer material described above.

Catheter 1 is made of the copolymer resin and has an outer diameter of 2.00 mm or less. The diameter of catheter 1 allows indwelling thereof in a blood vessel by using a 13G cannular having an inner diameter of 2.00 mm or more. Such a small-diameter catheter does not stimulate the wall surface of a blood vessel (e.g., the vena cava) having a larger diameter, thus obtaining a good antithrombotic property. The outer diameter of catheter 1 is preferably 1.7 mm or less, and the inner diameter thereof must be maximized. However, catheter 1 must have an appropriate thickness enough to maintain the physical properties thereof. Therefore, the inner diameter of catheter 1 is preferably larger than 40% and smaller than 80% of the outer diameter, more specifically, more than 0.5 mm and less than 1.4 mm. Catheter 1 must have flexibility for indwelling in a blood vessel.

Example

Thirty parts by weight of a polyurethane elastomer Pandex T-5265 (tradename) available from DAINIPPON INK & CHEMICALS, INC., 200 parts by weight of distilled water, 0.8 parts by weight of partially saponified polyvinyl alcohol Gosenol KH-17 (tradename) available from Nihon Gosei K.K., and 0.05 parts by weight of di-2-ethylhexylperoxydicarbonate were poured in a stainless autoclave having an internal volume of 10 ℓ. Internal air of the autoclave was substituted with $N_2$, and then 70 parts by weight of a vinyl chloride monomer were added in the autoclave, and the resultant mixture was suspension-polymerized at a temperature of 58°C for 15 hours. Nonreacted components were removed, and the residue was dried to obtain 90 parts by weight of a copolymer resin powder.

Subsequently, 0.5 parts by weight of calcium stearate and 0.1 part by weight of zinc stearate were mixed with respect to 100 parts by weight of the resultant copolymer resin mixture. The resultant mixture was molded by a PVC extrusion molding machine to obtain pellets at a temperature of 140°C to 160°C. By using the PVC extrusion molding machine again, a tube was extruded at a temperature of 150 to 180°C. The tube had an outer diameter of 1.5 mm and an inner diameter of 0.7 mm. The tube was transparent and flexible and could be thermally welded by using an RF welding machine.

The resultant tube was cut into pieces at a length of 19 cm, thereby preparing intravascular indwelling catheters.

Comparative Example

Fourty parts by weight of DEHP as a plasticizer were added to 60 parts by weight of PVC having a degree of polymerization (DP) of about 1,300 to prepare 100 parts by weight of a resin mixture. Subsequently, 0.5 parts by weight of calcium stearate and 0.1 part by weight of zinc stearate were mixed to the above mixture to cause a PVC extrusion molding machine to produce pellets at a temperature of 140°C to 160°C and then a tube at a temperature of 150 to 180°C. The tube had an outer diameter of 1.5 mm and an inner diameter of 0.7 mm. The tube was transparent and flexible and could be thermally welded by using an RF welding machine.

Test 1

The catheter of the example was compared with that of the comparative example according to the Chandeler loop method. According to this method, 89.5 µℓ of blood from a rabit's vein were quickly injected into the catheters without using an anti-coagulation agent, and open ends of the catheters were connected to constitute loops, respectively. The loops were rotated at a speed of 10 rpm, and times required for aggregating the blood in the tubes were measured. In this case, a ratio of the time required for the catheter of the example to that of the comparative example was calculated as follows:

$$\frac{\text{Example: Aggregation time}}{\text{Comparative example: Aggregation time}} = 1.8$$

Test 2

The catheters of the example and the comparative example were indwelled in a tibial vein of a crossbred dog for two weeks. The distal ends of the catheters reached near the merged portion between the above-mentioned vein and a subclavian vein. After two weeks, the tubes were removed to check conglutination of the catheters. No substance was found to be conglutinated on the catheter of the example by a naked eye. However, hemoagglutination caused by trapping of red blood corpuscles due to formation of a fibrin net was observed on the catheter of the comparative example. According to electron-microscopic observation, small amounts of blood platelets and corpuscles were agglutinated on the catheter of the example. However, a developed fibrin net was found on the catheter of the comparative example. A large number of red blood corpuscles were trapped in the fibrin net. Most of the trapped corpuscles were deformed.

As is apparent from the above description, the catheter according to the present invention can be easily indwelled in a blood vessel without problems. In addition, even if the catheter is indwelled in a large-diameter blood vessel such as the vena cava, the blood plasma wall is not stimulated, thus providing advantages such as a good antithrombotic property and stability of the physical properties for a long period of time.

Claims:

1. An antithrombotic material for medical use, which comprises a resin prepared by graft-polymerizing vinyl chloride with polyurethane resin.

2. An antithrombotic material according to claim 1, characterized in that the graft-polymerized resin is prepared by polymerizing, in an aqueous medium, a vinyl chloride monomer or a monomer mixture of a vinyl chloride monomer and a monomer copolymerizable therewith to prepare a homopolymer having a glass transition point of less than 30°C, in the presence of a thermoplastic polyurethane elastomer soluble with the vinyl chloride monomer and having a softening point of 20 to 100°C.

3. An antithrombotic material according to claim 1, characterized in that the graft-polymerized resin contains 10 to 80% by weight of the polyurethane elastomer.

4. An antithrombotic material according to claim 3, characterized in that a content of the polyurethane elastomer is 17 to 65% by weight.

5. An antithrombotic material according to claim 1, characterized in that the monomer copolymerizable with the vinyl chloride monomer to prepare the homopolymer having a glass transition point of less than 30°C is a material selected from the group consisting of olefins, vinylidene halides, vinyl esters, vinyl ethers, acrylic esters, and methacrylic esters.

6. An antithrombotic material according to claim 1, characterized in that a content of the monomer copolymerizable with the vinyl chloride monomer to prepare the homopolymer having a glass transition point of less than 30°C is not more than 50% by weight.

7. An antithrombotic material according to claim 1, characterized in that a content of the monomer copolymerizable with the vinyl chloride monomer to prepare the homopolymer having a glass transition point of less than 30°C is not more than 30% by weight.

8.    An intravascular indwelling catheter comprising a tube body which is made of a resin as defined in claims 1 to 7 and which has an outer diameter of not more than 2.0 mm.

9.    An intravenous holoneutrition catheter comprising a tube body which is made of a resin prepared by graft-polymerizing vinyl chloride with polyurethane resin and which has an outer diameter of not more than 2.0 mm.

10.    A central venous pressure measuring catheter comprising a tube body which is made of a resin prepared by graft-polymerizing vinyl chloride with polyurethane resin and which has an outer diameter of not more than 2.0 mm.

11.    An angiographic catheter comprising a tube body which is made of a resin prepared by graft-polymerizing vinyl chloride with polyurethane resin and which has an outer diameter of not more than 2.0 mm.

12.    An arterial pressure measuring catheter comprising a tube body which is made of a resin prepared by graft-polymerizing vinyl chloride with polyurethane resin and which has an outer diameter of not more than 2.0 mm.

0235482

# European Patent Office

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 512 452 (TOAGOSEI CHEMICAL INDUSTRY CO., LTD) * Claims 1-14,22-25,30; page 8, lines 28-35 * | 1-7 | A 61 L 33/00 A 61 L 29/00 C 08 F 283/00 // (C 08 F 283/00 C 08 F 214:06 ) |
| Y | | 8-12 | |
| X | CHEMICAL ABSTRACTS, vol. 101, no. 20, November 1984, page 61, abstract no. 172629g, Columbus, Ohio, US; & JP-A-59 93 712 (DAINIPPON INK AND CHEMICALS, INC) 30-05-1984 * Whole abstract * | 1-7 | |
| Y | Idem | 8-12 | |
| Y | EP-A-0 046 485 (SIEMENS) * Page 4, lines 29-36 * | 8-12 | **TECHNICAL FIELDS SEARCHED (Int. Cl.4)** A 61 L C 08 F A 61 M |
| Y | EP-A-0 102 685 (MALLINCKRODT INC.) * Page 6, lines 11-23 * | 8-12 | |

The present search report has been drawn up for all claims

| Place of search | Date of completion of the search | Examiner |
|---|---|---|
| THE HAGUE | 23-04-1987 | MEULEMANS R.A.M.G.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82